# EUROPEAN PATENT APPLICATION

(11) **EP 4 524 570 A1**
(43) Date of publication of application: **19.03.2025**
(21) Application number: 23196883.5
(22) Date of filing: 12.09.2023
(51) Int. Cl.: G01N 33/68

(54) **MEANS AND METHODS FOR DETERMINING THE POTENTIAL EXTENT OF BRAIN INJURY**

(71) Applicant: Klinikum der Universität München (LMU Klinikum) Anstalt des öffentlichen Rechtsvertreten durch den Ärztlichen Direktor un den Kaufm. Direktor, 81377 München (DE)
(72) Inventor: TIEDT, Steffen, 81377 München (DE); VLEGELS, Naomi, 81377 München (DE); KNUTH, Nicoló Luca, 81377 München (DE); DICHGANS, Martin, 81377 München (DE)
(74) Representative: Schiweck Weinzierl Koch Patentanwälte Partnerschaft mbB

(57) **Abstract**

The present invention generally relates to diagnosis of brain injury. More specifically, the invention relates to a method for determining the potential extent of brain injury in a subject, comprising quantifying in a blood sample obtained from said subject the level of BD-tau, wherein the higher the level of BD-tau in comparison to the level of BD-tau of a healthy subject is, the greater is the potential extent of brain injury of said subject.

## Description

### BACKGROUND

Evaluating brain injury is a fundamental aspect of clinical management of patients suffering from stroke. The extent of brain injury following stroke determines immediate clinical management and long-term patient outcomes. Early detection and continuous monitoring are paramount as timely interventions can mitigate complications and enhance recovery. In clinical routine, blood tests support the detection and monitoring of injuries of most organs, except for the brain.

Current diagnostic algorithms to assess brain injury in stroke primarily rely on neuroimaging techniques such as non-contrast imaging, CT perfusion, and MRI. While offering detailed insights into brain structures, their limitations include restricted availability in both resource-limited and well-equipped settings, unsuitability for certain patients, challenges associated with logistics including an elevated risk for complications in severely ill patients, high costs, and potential radiation exposure. Consequently, they may not be ideal for frequent monitoring of brain injury. Blood-based biomarkers, in contrast, are less invasive and have the potential for wider accessibility, particularly when assessed at the point-of-care. Additionally, conventional neuroimaging may not entirely capture the nuances of brain injury post-stroke, such as selective neuronal loss. In fact, infarct size derived from neuroimaging only accounted for less than 15% of the treatment effect from endovascular treatment (see, for example, Boers *et al.,* 2019).

Gonzales-Ortiz and colleagues established that a specific isoform of tau, termed herein as brain derived tau (BD-tau), can be used a biomarker of Alzheimer's disease by determining BD-tau levels in plasma/serum in a cohort of patients with Alzheimer's disease (Gonzalez-Ortiz, Dias, *et al.,* 2023). Furthermore, it was demonstrated that BD-tau levels in blood can be used as a proxy in evaluation of Alzheimer's disease-dependent neurodegenerative processes, based on correlation of blood BD-tau levels with histology (Gonzalez-Ortiz, Turton, *et al.,* 2023). In the same study, BD-tau was also investigated in a cohort of patients with stroke. BD-tau was also reported to be increased in these patients, however the authors stopped short of comparing BD-tau levels of patients to healthy controls, let alone drew any conclusion on the basis of the BD-tau values. Accordingly, BD-tau can only be regarded as increased within the cohort of patients with stroke, but no conclusion can be drawn as to the validity of BD-tau as a biomarker in acute stroke. Hence, the diagnostic value of a candidate biomarker must be warranted by sufficient data and meaningful comparison to healthy controls.

Previously studied biomarkers for brain injury, such as Neurofilament Light Chain (NfL), neuron-specific enolase (NSE), glial fibrillary acidic protein (GFAP), and S 100 calcium-binding protein B (S100B) either lacked specificity for the brain or failed to gauge the extent of brain injury sufficiently early. Recently, the tubulin associated unit (tau) protein, has been the subject of intense research in the neurodegeneration field and put forth as a potential biomarker for brain injury. While increased levels of overall tau, termed herein as total-tau (t-tau), have been reported in CSF of patients with acute stroke (Ki 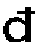 emet-Piskač *et al.,* 2018), t-tau concentration in serum has not been found to serve as a suitable biomarker for acute stroke (Gonzalez-Ortiz, Turton, *et al.,* 2023). This demonstrates that the relationship between the concentration of a biomarker in CSF and blood is often not straightforward, and a CSF biomarker of brain injury can thus not be reasonably extrapolated to a blood biomarker for the use in routine diagnostics.

As said, imaging methods which provide the basis for scoring systems such as Alberta Stroke Program Early CT Score (ASPECTS) or collateral grade are poorly available and cannot be used continuously. Although that makes a blood-based biomarker for the diagnosis of brain injury desired and valuable, there is thus far no diagnostic test available for evaluating the extent of brain injury, e.g. after stroke. A blood-based biomarker is therefore needed in the art to allow for determining the potential extent of brain injury in a timely and cost-effective fashion.

### SUMMARY OF THE INVENTION

The present invention therefore addresses this need and provides a solution as described herein, shown in the examples, illustrated in the figures and as defined in the claims.

In a first aspect, the present invention relates to a method for determining the potential extent of brain injury in a subject, comprising quantifying in a blood sample obtained from said subject the level of BD-tau, wherein the higher the level of BD-tau in comparison to the level of BD-tau of a healthy subject is, the greater is the potential extent of brain injury of said subject.

In one embodiment, said condition causing brain injury is stroke, preferably ischemic stroke, e.g. acute ischemic stroke.

In another embodiment, a level of BD-tau of about 1.8 pg/ml or more is indicative of the potential extent of brain injury of said subject.

In a second aspect, the present invention relates to a method for determining the progression of the potential extent of brain injury in a subject, comprising quantifying in a blood sample obtained from said subject over the course of time the level of BD-tau, wherein an increase in the level of BD-tau over the course of time, is indicative of the progression of the potential extent of brain injury of said subject.

In one embodiment of the second aspect, said subject is further stratified:
(i) wherein an increase in the level of BD-tau of about 0.9 pg/ml/h or higher is equivalent to an ASPECTS of 0-2,
(ii) wherein an increase in the level of BD-tau between about 0.5 to 0.9 pg/ml/h is equivalent to an ASPECTS of 3-5,
(iii) wherein an increase in the level of BD-tau between about 0.1 to 0.5 pg/ml/h is equivalent to an ASPECTS of 6-8,
(iv) wherein an increase in the level of BD-tau up to about 0.1 pg/ml/h is equivalent to an ASPECTS of 9-10,
wherein the stratification allows for the selection of said subject for an appropriate treatment and/or the prediction of outcome.

In another embodiment of the second aspect, said subject is further stratified:
(i) wherein an increase in the level of BD-tau of about 0.9 pg/ml/h or higher is equivalent to a collateral grade 0,
(ii) wherein an increase in the level of BD-tau between about 0.5 to 0.9 pg/ml/h is equivalent to a collateral grade 1,
(iii) wherein an increase in the level of BD-tau between about 0.1 to 0.5 pg/ml/h is to a collateral grade 2,
(iv) an increase in the level of BD-tau up to about 0.1 pg/ml/h is equivalent to a collateral grade 3,
wherein the stratification allows for the selection of said subject for an appropriate treatment and/or the prediction of outcome.

In another aspect, the present invention relates to a use of the level of BD-tau quantified in a blood sample obtained from a subject suspected to or suffering from a condition causing brain injury for determining the potential extent of brain injury in said subject.

In still another aspect, the present invention relates to a use of the increase in the level of BD-tau quantified in a blood sample obtained from a subject suspected to or suffering from a condition causing brain injury for determining the progression of the potential extent of brain injury in a subject.

In yet another aspect, the present invention relates to a kit comprising an antibody specific for BD-tau and at least two containers for storing and/or processing blood samples.

### BRIEF DESCRIPTION OF THE DRAWINGS

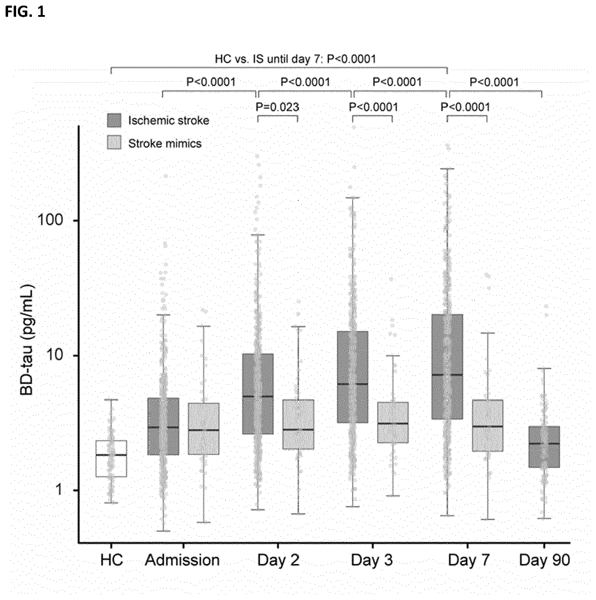

**Figure 1****. Temporal course of plasma BD-tau levels after stroke onset**
   Plasma levels of BD-tau in patients with ischemic stroke over time in comparison to patients with stroke mimics and healthy controls. P values were calculated using a linear mixed model (to compare time points within the stroke cohort) and analysis of variance with post-hoc Tukey HSD tests (to compare patients with ischemic stroke with healthy controls and stroke mimics).

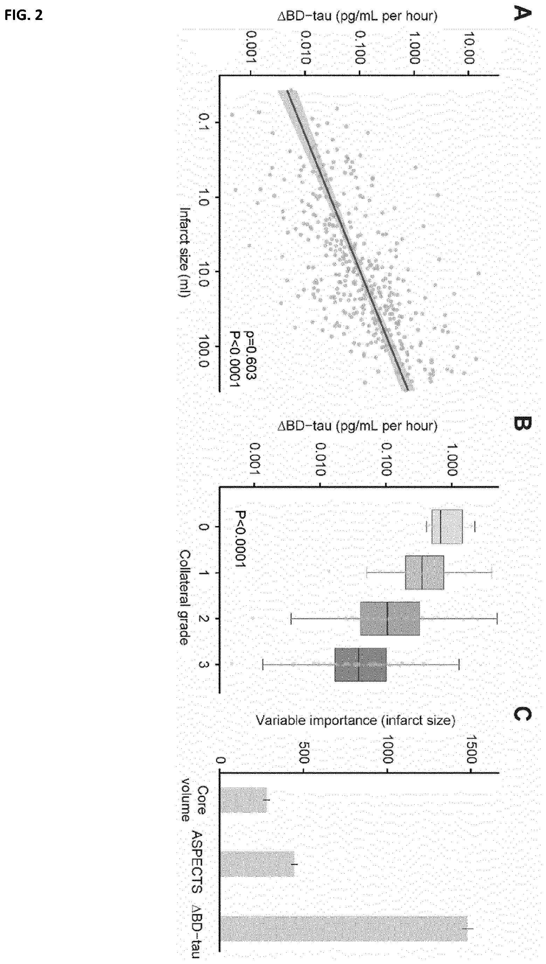

**Figure 2****. Relation of ΔBD-tau with imaging-based metrics of brain injury**
   (A) ΔBD-tau in relation to infarct size determined by delayed neuroimaging. The P value was calculated using Spearman's rank correlation. (B) ΔBD-tau in relation to the degree of collateral supply. The P value was calculated using one-way analysis of variance. (C) Variable importance to predict infarct size in a multivariable random forest regression model. Shown are the median and 5% and 95% quantiles of importance values. ΔBD-tau, change of BD-tau between admission and day 2 adjusted for the time passed in hours; ASPECTS, Alberta Stroke Program Early CT Score.

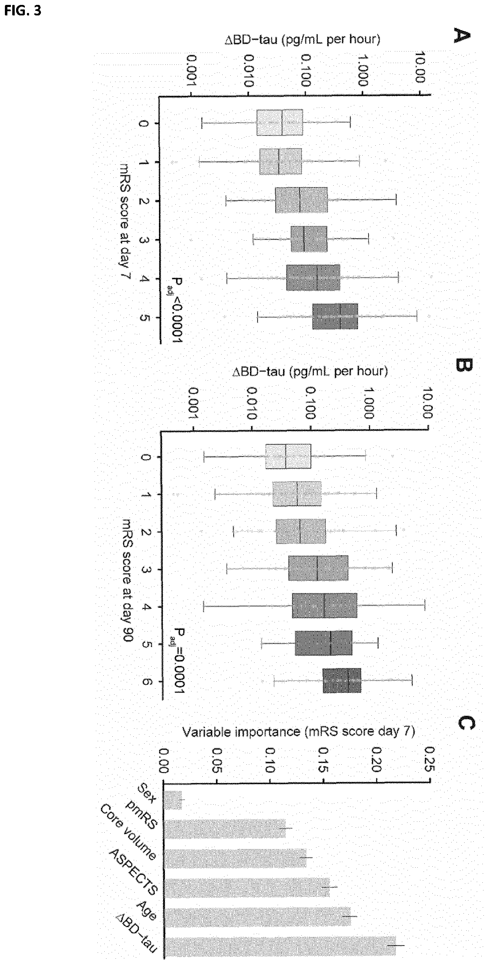

**Figure 3****. Relation of ΔBD-tau with functional outcome**
   (A) ΔBD-tau in relation to functional outcome at day 7. The P value was calculated using a multivariable logistic regression model adjusting for age, sex, hypertension, and the premorbid mRS score. (B) ΔBD-tau in relation to functional outcome at day 90. The P value was calculated using a multivariable logistic regression model adjusting for age, sex, hypertension, and the premorbid mRS score. (C) Variable importance to predict functional outcome at day 7 in a multivariable random forest regression model. Shown are the median and 5% and 95% quantiles of importance values. mRS, modified Rankin Scale; pmRS, premorbid modified Rankin Scale.

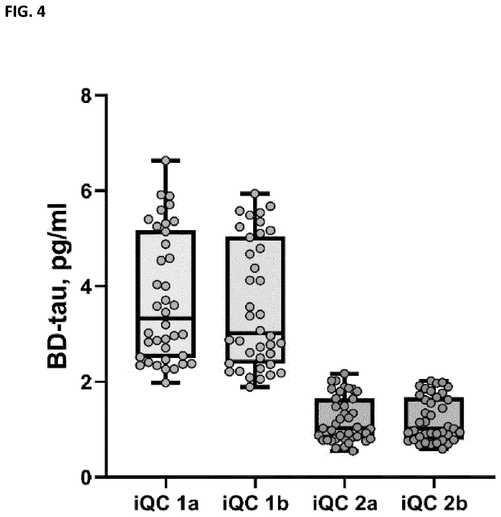

**Figure 4****. Concentrations of plasma BD-tau in internal quality controls**
   Variation intra and inter-runs was assessed using two internal quality controls (iQC; 1 and 2) at the beginning (a) and the end of each plate (b). Mean concentration for iQC 1 (n=76) was 3.7±1.32 pg/ml with a CV of 8.5% in the whole cohort. Mean concentration for iQC 2 (n=78) was 1.2±0.46 with a CV of 9.1% in the whole cohort. CV, coefficient of variation.

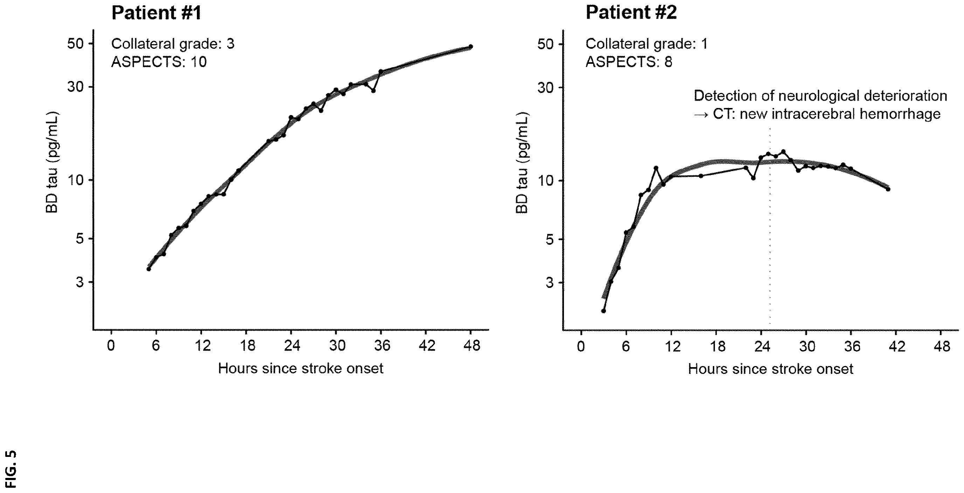

**Figure 5****. Real-time evolution of BD-tau levels in the first 48 hours after stroke onset in two patients**
   Shown are serial high-frequency assessments of BD-tau levels of two patients in the first 48 hours after stroke onset. Plasma samples were collected hourly, except between 3AM and 5AM in the morning and during diagnostic procedures. The black dots and line indicate the raw values. The red line indicates the fit derived from unbiased local polynomial regression. ASPECTS was assessed on admission non-contrast computerized tomography. The collateral grade was assessed on CT angiography with a higher number indicating better collateral supply.

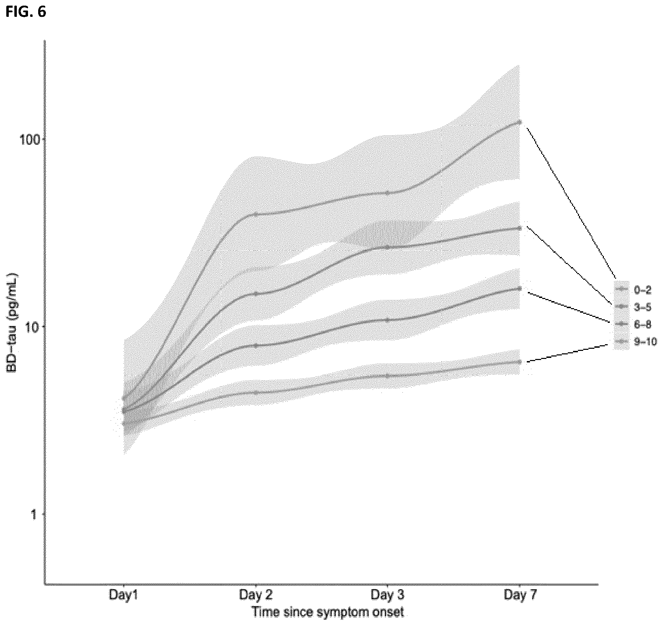

**Figure 6****. Development of BD-tau levels over time after stroke stratified by ASPECTS**
   Shown is the development of BD-tau over days since symptom onset stratified by ASPECTS upon admission. A higher ASPECTS represents less severe stroke severity on admission CT. Grey areas indicate 95% confidence intervals.
**Figure 7****. Variable importance for ΔBD-tau in comparison to absolute BD-tau levels upon admission and at day 2**
   Shown are the median and 5% and 95% quantiles of importance values to predict BD-tau levels at day 1 (upper panel) and ΔBD-tau (lower panel) in a multivariable random forest regression model. GFR, glomerular filtration rate.
**Figure 8****. ΔBD-tau stratified by ASPECTS upon admission**
   Shown is ΔBD-tau stratified by ASPECTS upon admission. A higher ASPECTS represents less severe stroke severity on admission CT. The P values were calculated using a Kruskal-Wallis test (lower left corner) and post-hoc Dunn Tests (top).
**Figure 9****. Relation of ΔBD-tau with ischemic core volume upon admission**
   Shown is the association between ΔBD-tau and CT perfusion-based ischemic core volume upon admission. The P value was calculated using a linear regression model. The linear fit is shown in blue. The 95% confidence interval is shown in grey.

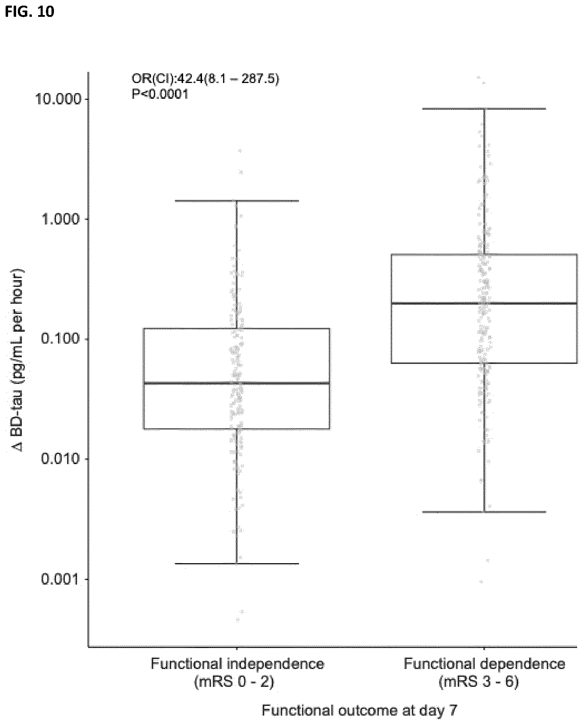

**Figure 10****. ΔBD-tau and status of functional independence at day 7**
   Shown are boxplots representing ΔBD-tau comparing patients with stroke with functional independence (mRS score 0-2) at day 7 with those that were functionally dependent (mRS score 3-6). The boxes represent (from bottom to top) the first quartile, the median and the third quartile. The upper whiskers indicate the third quartile plus 1.5 times the interquartile range while the lower whiskers indicate the first quartile minus 1.5 times the interquartile range. The odds ratio (95% confidence interval) and P value were calculated using a multivariable logistic regression model adjusting for age, sex, hypertension, and the premorbid mRS score. OR, Odds ratio; Cl, Confidence Interval.

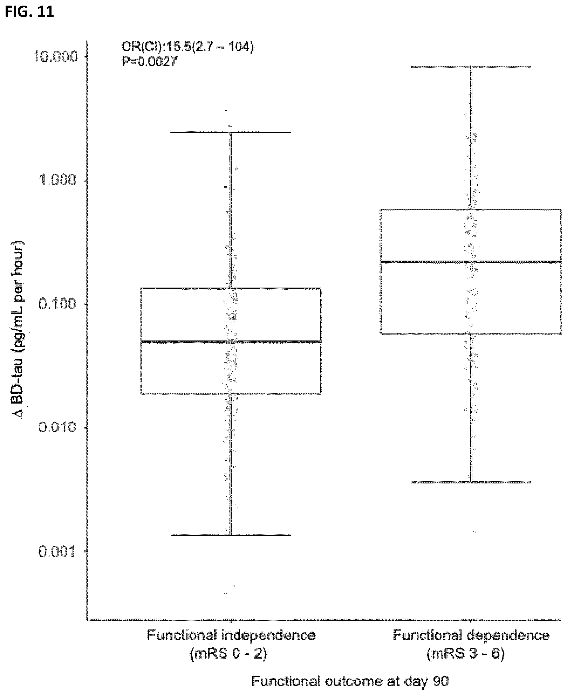

**Figure 11****. ΔBD-tau and status of functional independence at day 90**
   Shown are boxplots representing ΔBD-tau comparing patients with stroke with functional independence (mRS score 0-2) at day 90 with those that were functionally dependent (mRS score 3-6). The boxes represent (from bottom to top) the first quartile, the median and the third quartile. The upper whiskers indicate the third quartile plus 1.5 times the interquartile range while the lower whiskers indicate the first quartile minus 1.5 times the interquartile range. The odds ratio (95% confidence interval) and P value were calculated using a multivariable logistic regression model adjusting for age, sex, hypertension, and the premorbid mRS score.

### DETAILED DESCRIPTION

The finding of the present inventors therefore paves the way for stratifying subjects suffering from brain injury, such as acute ischemic stroke, whereby said subjects are stratified into categories with different clinical relevance. Notably, the present invention teaches how to quickly select patients for administration of specific treatments based on said stratification, which enables appropriate treatment in the critical time window following stroke. Importantly, appropriate treatment of acute ischemic stroke administered in a timely fashion can dramatically improve the functional outcome and even have lifesaving consequences. Furthermore, the adaptability of blood BD-tau levels for regular monitoring and potentially broader accessibility could thus streamline stroke assessment and make it more precise, thereby complementing existing neuroimaging-based algorithms. BD-tau seems thus to have the potential to revolutionize the assessment of potential brain injury extent in subjects with suspected brain injury or in conditions causing such brain injury.

Accordingly, the present invention provides BD-tau as a new blood-based biomarker which is suitable to determine the potential extent of brain injury in a subject with suspected brain injury. The inventors of the present invention showed that BD-tau reflects the dynamic evolution, e.g. in the acute phase of ischemic stroke and predicts, inter alia, the final infarct volume as well as the functional outcome of patients with stroke. The inventors of the present invention have surprisingly found that BD-tau outperforms currently used imaging tools in their predictive power of the final infarct size and functional outcome (see Fig. 2C), thus providing evidence for the concept that circulating brain-derived molecules detected by single-molecule assays are more precise in detecting and monitoring the extent of brain injury compared to neuroimaging.

It must be noted that as used herein, the singular forms "a", "an" and "the" include plural references and vice versa unless the context clearly indicates otherwise. Thus, for example, a reference to "a method" includes one or more of such methods, and a reference to "the method" includes equivalent steps and methods that could be modified or substituted known to those of ordinary skill in the art. Similarly, for example, a reference to "methods" includes "a method", respectively.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term". For example, A, B and/or C means A, B, C, A+B, A+C, B+C and A+B+C.

Throughout this specification and the claims or items, unless the context requires otherwise, the word "comprise" and variations such as "comprises" and "comprising" will be understood to imply the inclusion of a stated integer (or step) or group of integers (or steps). It does not exclude any other integer (or step) or group of integers (or steps). When used herein, the term "comprising" can be substituted with "containing", "composed of", "including", "having" or "carrying". When used herein, "consisting of" excludes any integer or step not specified in the claim/item. When used herein, "consisting essentially of" does not exclude integers or steps that do not materially affect the basic and novel characteristics of the claim/item. In each instance herein any of the terms "comprising", "consisting essentially of" and "consisting of" may be replaced with either of the other two terms.

The term "about" or "approximately" as used herein in the context of the value of BD-tau concentration in a plasma/serum sample means within 60%, preferably within 40%, and more preferably within 20% of the given value. It includes also the concrete number, e.g., about 5 includes 5.

The term "or less" or "less than", or the term "or more" or "more than" includes the concrete number. For example, less than 5 means ≤5 and more than 5 means ≥5.

It should be understood that this invention is not limited to the particular methodology, protocols, material, reagents, and substances, etc., described herein. The terminologies used herein are for the purpose of describing particular embodiments only and are not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

Accordingly, the present invention relates to a method for determining the potential extent of brain injury in a subject, comprising quantifying in a blood sample obtained from said subject the level of BD-tau, wherein the higher the level of BD-tau in comparison to the level of BD-tau of a healthy subject is, the greater is the potential extent of brain injury of said subject.

The term "subject" includes all mammals, but is not limited to mouse, rat, dog, horse, camel, primates, etc., primates being preferred and human beings being most preferred. In a preferred embodiment the subject is suspected/assumed to comprise or already comprises elevated levels of BD-tau. When used herein, the term "subject" is equivalently used with the term "patient". Hence these two terms can be interchangeably used herein. The term "subject" as used herein includes both non-adult and adult human subjects.

A "healthy" subject when referred to herein means that the subject does not suffer or is not suspected to suffer from a condition causing brain injury. Such a healthy subject provides for a reference value of BD-tau. The person skilled in the art is familiar with the concept of reference values (or "normal values") In particular, the term reference value may relate to the actual value of the level in one or more control samples or it may relate to a value derived from the actual level in one or more control samples. Preferably, samples of at least 50, more preferably at least 100, more preferably at least 500, most preferably at least 1000 subjects.

In the simplest case, the reference value is the same as the level measured in the control sample or the average of the levels measured in a multitude of control samples. However, the reference value may also be calculated from more than one control sample. E.g., the reference value may be the arithmetic average of the level in control samples representing the control status (e.g. healthy state). Preferably, the reference value relates to a range of values that can be found in a plurality of comparable control samples, e.g. the average one or more times the standard deviation. Similarly, the reference value may also be calculated by other statistical parameters or methods, for example as a defined percentile of the level found in a plurality of control samples, e.g. a 90%, 95%, 97.5%, or 99% percentile.

According to the method of the present invention, said blood sample preferably contains serum or plasma.

As used herein, the term "blood sample" refers to a biological sample derived from blood, preferably peripheral (or circulating) blood. The blood sample can be whole blood, plasma or serum, although plasma is typically preferred. Since the blood sample is obtained from a subject, this means that the methods of diagnosis of the present invention are carried out *in vitro* or *ex vivo.* A blood sample contains both serum and plasma or either serum or plasma.

The definition of "sample" also includes samples that have been manipulated in any way after their procurement, such as by centrifugation, filtration, precipitation, dialysis, chromatography, treatment with reagents, washed, or enriched for certain proteins, such as BD-tau.

As used herein, the term "brain injury" refers to any injury occurring in the brain of a living organism. Brain injury can be broadly categorized into two main types: traumatic brain injury (TBI) and acquired brain injury caused by endogenous mechanisms. These categories encompass a wide range of specific injuries and conditions. TBI occurs when an external force traumatically injures the brain in a sudden physical impact or trauma to the head. It can result from accidents, falls, or blows to the head and may range from mild concussions to severe injuries. TBIs can lead to various symptoms, including headaches, memory problems, and changes in behavior. On the other hand, acquired brain injury refers to damage to the brain that occurs after birth due to non-traumatic causes. This category includes conditions like strokes, brain tumors, infections, lack of oxygen, and toxic substances exposure. Acquired brain injuries can lead to a wide range of neurological impairments depending on the underlying cause.

Likewise, though a patient may have a brain injury which has a certain extent, the extent does not necessarily become greater or may progress. Accordingly, the term "potential" implies that the methods of diagnosis of the present invention provide predictions as to whether or not the extent of brain injury may increase or progress, but - self-explanatory as it is - cannot provide a 100% safe prediction, since, apart from the level of BD-tau or the increase of the level of BD-tau individual factors such as sex, age, weight, nutritional status, health status, premedication etc. may have an influence on the extent of brain injury.

As used herein, tau protein refers to a protein abundant in neurons of the central nervous system, particularly in the cerebral cortex. Tau proteins are a group of six highly soluble protein isoforms produced by alternative splicing of the gene *MAPT.* It is believed that one of the main functions of tau is to stabilize microtubules in axons and dendrites.

Tau protein has received much attention in the context of certain neurodegenerative diseases, such as Alzheimer's disease, frontotemporal dementia, and other tauopathies. According to the main hypothesis underlying pathophysiology of these diseases is that tau, highly soluble under normal conditions, in its hyperphosphorylated form misfolds and becomes insoluble. Misfolded tau aggregates into filamentous structures and neurofibrillary tangles, wherein these tau aggregates cause toxicity through its accumulation inside cells, ultimately leading to neuronal cell death and cognitive decline.

The observation that t-tau concentrations in plasma samples of patients with Alzheimer's disease do not correlate with t-tau concentrations in CSF samples from the same patients suggested that plasma and CSF t-tau do not originate from the same tissue sources. Indeed, while tau is known to be highly abundant in the CNS, the protein is also present in peripheral tissues (e.g. liver, kidney, heart). The protein structure of tau in the brain and the periphery has fundamental differences in splice variants: the main form of tau in the periphery is distinguishable from isoforms found in the central nervous system by the presence of a large peptide insert resulting from the transcription of an extra exon (exon 4a) of the MAPT gene. Thus, the term "BD-tau" as used herein refers to brain-derived tau protein, which can be selectively measured in blood by targeting an epitope found specifically on the tau isoform originating from the brain.

BD-tau as referred to herein corresponds to tau 2N4R isoform comprising 441 amino acid residues, also referred to as tau-F or tau-4, with the UniProtKB entry P10636-8. BD-tau as used herein includes the full-length protein and any fragment thereof or allelic variant thereof as long as these are distinguishable from other tau proteins.

The level of BD-tau may be quantified as described in Gonzalez-Ortiz, Turton, *et al.,* 2023 (see page 3 paragraph 4, and page 4 paragraphs 3 and 4), or as described in the examples herein.

Furthermore, the present invention relates to a method for determining the progression of the potential extent of brain injury in a subject, comprising quantifying in a blood sample obtained from said subject over the course of time the level of BD-tau, wherein an increase in the level of BD-tau over the course of time, is indicative of the progression of the potential extent of brain injury of said subject.

As used herein, "progression" refers to the development or advancement of a particular condition or state, specifically the potential extent of brain injury in a subject. It implies that there is a change or increase in the severity or extent of the brain injury over a period of time. In the context of the present invention, "progression" indicates that the condition is getting worse or more severe as time passes, as evidenced by an increase in the level of BD-tau in the blood sample.

According to the present invention, said brain injury is preferably an acute brain injury.

As used herein, term "acute brain injury" refers to a medical condition characterized by the sudden and often severe damage to brain tissue. Acute brain injuries encompass a range of conditions, including traumatic brain injury, ischemic stroke, hemorrhages and infections. These injuries typically require immediate medical attention to assess and mitigate the extent of damage and initiate appropriate interventions for optimal patient outcomes.

According to the present invention, said subject is preferably suspected to or suffers from a condition causing brain injury.

Suspected cases of brain injury or conditions thereof may arise from a range of incidents, including accidents involving head injuries and cases with symptoms consistent with brain injury. When a brain injury is suspected, it is crucial to conduct a thorough examination to establish the correct diagnosis of brain injury and the extent thereof. The method of the present invention can thus conveniently be used to determine the appropriate treatment and assess whether the patient can be safely discharged following admission with suspected brain injury.

According to the present invention, said condition causing brain injury is preferably a stroke, preferably ischemic stroke, e.g. acute ischemic stroke.

As used herein, "stroke" refers to a form of acquired brain injury, wherein the damage to the brain occurs following a vascular event. Principally, there are two main types of stroke: hemorrhagic stroke and ischemic stroke. Hemorrhagic strokes occur when a blood vessel in the brain ruptures, causing bleeding within or around the brain. There are two further subtypes of hemorrhagic strokes: intracerebral and subarachnoid hemorrhage. Intracerebral hemorrhage results from bleeding within the brain tissue itself, often due to the rupture of a weakened blood vessel. Subarachnoid hemorrhages occur when there is bleeding into the space between the brain and the thin tissue that covers it (the arachnoid membrane). This is often caused by the rupture of an aneurysm or other blood vessel abnormalities.

Ischemic strokes are the most common type of strokes, accounting for about 87% of all strokes, which occur when a blood clot or plaque buildup in an artery reduces or completely blocks blood flow to a part of the brain. There are two further subtypes of ischemic strokes: a thrombotic stroke and embolic stroke. Thrombotic stroke occurs when a blood clot (thrombus) forms in an artery supplying blood to the brain, typically in arteries already narrowed by atherosclerosis (plaques). Embolic stroke occurs when a blood clot or debris forms elsewhere in the body (often the heart) and travels through the bloodstream until it gets lodged in a smaller brain artery and disrupts the blood flow.

An acute ischemic stroke is a medical condition characterized by a sudden interruption of blood flow to a specific area of the brain, typically caused by the occlusion of a cerebral artery due to a blood clot or other blockage. This interruption leads to a rapid onset of neurological symptoms, such as weakness, speech difficulties, and loss of coordination. Timely intervention, often involving thrombolytic medications or mechanical clot retrieval techniques, is vital to restore blood flow and minimize brain damage in cases of acute ischemic stroke. Thus, the method of the present invention is particularly useful in determining the extent of infarction area for these interventions as well as in predicting the time course of the brain damage.

According to the method of the present invention, the level of BD-tau is preferably quantified over the course of time.

As used herein, the phrase "course of time" refers to a period or duration during which something is observed, measured, or monitored. In this context, it signifies that the assessment or quantification of the level of BD-tau in a blood sample is conducted repeatedly over a span of time. Essentially, it means that measurements are taken at multiple points in time to track changes or developments in the level of BD-tau, allowing for the assessment of the progression of the brain injury in the subject. Course of time in the context of this application may be, for example and without limitation, 0.5, 1, 2, 3, 6, 12, 18, 24, 30, 36, 42, and 48 hours.

According to the method of the present invention, preferably a level of BD-tau of about 1.8 pg/ml or more is indicative of the potential extent of brain injury of said subject.

According to the method of the present invention, preferably an increase of about 0.01 pg/ml/h or more is indicative of the potential progression of the extent of brain injury.

According to the method of the present invention, the course of time is preferably at least about 0.5 hour.

According to the method of the present invention, the quantification of the level of BD-tau is preferably at least within 48 hours after onset of symptoms.

According to the method of the present invention, said subject is preferably further stratified, wherein an increase in the level of BD-tau of about 0.9 pg/ml/h or higher is equivalent to an ASPECTS of 0-2, wherein an increase in the level of BD-tau between about 0.5 to 0.9 pg/ml/h is equivalent to an ASPECTS of 3-5, wherein an increase in the level of BD-tau between about 0.1 to 0.5 pg/ml/h is equivalent to an ASPECTS of 6-8, wherein an increase in the level of BD-tau up to about 0.1 pg/ml/h is equivalent to an ASPECTS of 9-10, and wherein the stratification allows for the selection of said subject for an appropriate treatment and/or the prediction of outcome.

As used herein, the term "ASPECTS", abbreviated from Alberta stroke program early CT score, refers to a 10-point quantitative topographic CT scan score used for middle cerebral artery stroke patients. The ASPECTS is a mortality predictor and its value correlates inversely with the severity and evolution of patients. See, for example, Barber *et al.* (2000). ASPECTS is quantified as follows: segmental estimation of the middle cerebral artery (MCA) vascular territory is made, and 1 point is deducted from the initial score of 10 for every region involved: caudate, putamen, internal capsule, insular cortex, M1: 'anterior MCA cortex', corresponding to frontal operculum; M2: 'posterior MCA cortex', corresponding to posterior temporal lobe; M4: 'anterior MCA territory immediately superior to M1', M5: 'lateral MCA territory immediately superior to M2'; M6: 'posterior MCA territory immediately superior to M3'. For example, an ASPECTS of ≤7 predicts a worse functional outcome at 3 months and identifies stroke patients unlikely to make an independent recovery despite thrombolytic treatment.

According to the method of the present invention, said subject is preferably further stratified, wherein an increase in the level of BD-tau of about 0.9 pg/ml/h or higher is equivalent to a collateral grade 0, wherein an increase in the level of BD-tau between about 0.5 to 0.9 pg/ml/h is equivalent to a collateral grade 1, wherein an increase in the level of BD-tau between about 0.1 to 0.5 pg/ml/h is to a collateral grade 2, an increase in the level of BD-tau up to about 0.1 pg/ml/h is equivalent to a collateral grade 3, wherein the stratification allows for the selection of said subject for an appropriate treatment and/or the prediction of outcome.

As used herein, the term "collateral grade" refers to the assessment of collateral circulation in the brain. Collateral circulation refers to the alternative pathways through which blood can flow to the brain when the primary blood vessels are occluded during an ischemic stroke. The degree of collateral supply through alternative pathways including the leptomeningeal vessel is important and correlates with the presence of smaller final infarct volume. Collateral grade predicts final infarct size but does not independently predict clinical outcome. Typically, collateral grade ranges from 0 to 3, wherein grade 0 means absent collateral supply to the occluded MCA territory; collateral grade 1 means collateral supply filling between 0% and 50% of the occluded MCA territory; collateral grade 2 means collateral supply filling between 50% and 100% of the occluded MCA territory; and collateral grade 3 means collateral supply filling 100% of the occluded territory.

According to the method of the present invention, said treatment is preferably intravenous thrombolysis, endovascular thrombectomy, and/or administration of a suitable agent.

As used herein, "intravenous thrombolysis" refers to a medical procedure that entails the administration of thrombolytic agents, administered either through systemic administration or local release by a catheter at the clot site. See, for example, Baig and Bodle (2023). This class of medicine is used in acute myocardial infarction, deep vein thrombosis, pulmonary embolism, acute ischemic stroke, acute peripheral arterial occlusion, occlusion of indwelling catheters, and intracardiac thrombus formation in order to dissolve dangerous intravascular clots and prevent ischemic damage by improving blood flow. Examples of thrombolytic agents include, but are not limited to, the following: streptokinase, alteplase, reteplase, tenecteplase, urokinase, prourokinase, and anistreplase. All available thrombolytic agents are plasminogen activators, i.e. possessing serine protease activity that cleave plasminogen into active plasmin and thus leading to lysis of fibrin clots.

Adverse effects of most fibrinolytic agents include, but are not limited to, the following: bleeding, hypotension, allergic reactions, angioedema, anaphylactic shock, cholesterol embolism, and reperfusion arrhythmias. Bleeding is the most frequent complication of thrombolytic therapy and can occur in puncture sites or spontaneously anywhere inside the body. Intracranial hemorrhage is the greatest concern. Risk factors associated with hemorrhagic complications include elderly patients, uncontrolled hypertension, recent stroke or surgery, the presence of bleeding diathesis, and concurrent use of anticoagulants.

As used herein, "endovascular thrombectomy" refers to a medical procedure used to remove blood clots from within blood vessels, in the context of treating acute ischemic stroke or other conditions where blood clots obstruct blood flow to vital organs. See, for example, Oliveira, Viana and Santos (2022). The procedure typically consists of inserting a thin, flexible catheter into a major artery and angiography-guided navigation to the clot site. A thrombectomy device contained at the tip of the catheter is then used to mechanically retrieve the blood clot from the occluded area. Endovascular thrombectomy is more effective the earlier it is performed after stroke symptoms onset, it is therefore essential that eligible patients are treated as soon as possible after stroke.

While endovascular thrombectomy is considered highly effective in treating thrombosis underlying acute ischemic stroke, it is also associated with serious complications, such as symptomatic intracerebral bleeding with potentially fatal consequences.

Thus, intravenous thrombolysis and endovascular thrombectomy are associated with considerable risks, and should therefore be performed only in cases when justified by an appropriate diagnosis, e.g. acute ischemic stroke with a limited ischemic area. The method of the present invention is particularly useful in addressing this issue, in that it can identify patients suffering from acute ischemic stroke within the critical time window when said subject would benefit the most from said treatment.

According to the method of the present invention, said suitable agent is preferably a neuroprotective agent.

As used herein, "neuroprotective agent" refers to a drug or a substance with neuroprotective effects. Neuroprotective effect refers to a treatment or intervention aiming at preventing neuronal loss and neurodegeneration through inhibition of pathophysiological pathways and processes that are otherwise injurious to the nervous system. Neuroprotective agents may act, for example, through inhibition of inflammatory processes and apoptosis, attenuation of oxidative stress and reduction of free radicals. Several neuroprotective agents are used to treat other conditions as well. Drugs such as anti-Alzheimer's, anti-Parkinson's, and anti-ischemics are neuroprotective agents.

Currently, the US Food and Drug Administration (FDA) has not yet approved a specific treatment modality as neuroprotective therapy for ischemic stroke. However, there are many ongoing clinical trials with promising results, and a neuroprotective agent for specific use in acute ischemic stroke is expected to become available in the near future (Ghozy *et al.,* 2022).

Furthermore, the present invention also relates to use of the level of BD-tau quantified in a blood sample obtained from a subject suspected to or suffering from a condition causing brain injury for determining the potential extent of brain injury in said subject.

According to the present invention, preferably the higher the level of BD-tau in comparison to the level of BD-tau of a healthy subject is, the greater is the potential extent of brain injury of said subject.

Furthermore, the present invention also relates to use of the increase in the level of BD-tau quantified in a blood sample obtained from a subject suspected to or suffering from a condition causing brain injury for determining the progression of the potential extent of brain injury in a subject.

According to the present invention, preferably an increase in the level of BD-tau over the course of time is indicative of the progression of the potential extent of brain injury of said subject.

Furthermore, the present invention also relates to a kit comprising an antibody specific for BD-tau and at least two containers for storing and/or processing blood samples. Said antibody specific to BD-tau may be a commercial TauJ.5H3 antibody (Bioventix Plc.), which specifically binds to BD-tau in a concentration-dependent manner. The TauJ.5H3 antibody recognizes tau isoforms that are derived from the CNS but avoids tau forms that include the exon 4a region predominantly expressed in peripheral tissues.

The kit of the present invention is preferably used in the method of the invention. Said kit is preferably a diagnostic kit. Said kit may further comprise means for administering said antibody, such as a syringe, a reaction container, and the like. Said kit of the invention may further comprise a recombinant full-length tau to be used as the assay calibrator.

The kit of the present invention preferably further comprises an antibody directed against N-terminal tau. Preferably, the N-terminal tau-specific antibody is a detection antibody. The detection antibody may have been raised in any vertebrate species including, without limitation, primates, mice, rats, goats, chickens, rabbits, donkeys, and the like and may be specific to immunoglobulins such as, without limitation, IgA, IgG, IgM, IgE or IgD immunoglobulins or may be pan-specific. More preferably, the detection antibody is a mouse monoclonal antibody raised against N-terminal region of tau. The detection antibody may be further conjugated to a detectable label, wherein detectable label may be any appropriate chemical substance or enzyme, which directly or indirectly generates a detectable compound or signal in a chemical, physical, optical, or enzymatic reaction. For example, a fluorescent label can be conjugated to the detection antibody to generate fluorescence as detectable signal. Such antibodies are available commercially and can be made by those skilled in the art.

In cases where the detection antibody is not conjugated to a detectable label, the kit may further comprise the step of contacting the detection antibody with a further specific binding partner, such as a further antibody that specifically binds to the detection antibody. The further specific binding partner may comprise a detectable label, such as a fluorescent label that can be used for the detection. Presence and/or amount of the BD-tau in plasma or serum sample can thus be detected or determined by measuring or observing a reporter signal obtained from a detectable label comprised in the detection antibody or further specific binding partner.

The present invention may be summarized in the following items:
Item 1: a method for determining the potential extent of brain injury in a subject, comprising quantifying in a blood sample obtained from said subject the level of BD-tau, wherein the higher the level of BD-tau in comparison to the level of BD-tau of a healthy subject is, the greater is the potential extent of brain injury of said subject.
Item 2: a method for determining the progression of the potential extent of brain injury in a subject, comprising quantifying in a blood sample obtained from said subject over the course of time the level of BD-tau, wherein an increase in the level of BD-tau over the course of time, is indicative of the progression of the potential extent of brain injury of said subject.
Item 3: the method of any one of the preceding items, wherein said brain injury is acute brain injury.
Item 4: the method of any one of the preceding items, wherein said subject is suspected to or suffers from a condition causing brain injury.
Item 5: the method of any one of the preceding items, wherein said condition causing brain injury is stroke, preferably ischemic stroke, e.g. acute ischemic stroke.
Item 6: he method of any one of the preceding items, wherein the level of BD-tau is quantified over the course of time.
Item 7: the method of any one of the preceding items, wherein a level of BD-tau of about 1.8 pg/ml or more is indicative of the potential extent of brain injury of said subject.
Item 8: the method of any one of items 2 to 7, wherein an increase of about 0.01 pg/ml/h or more is indicative of the potential progression of the extent of brain injury.
Item 9: the method of any one of items 2 to 8, wherein the course of time is at least about 0.5 hour.
Item 10: the method of any one of the preceding items, wherein the quantification of the level of BD-tau is at least within 48 hours after onset of symptoms.
Item 11: the method of any one of items 2 to 10, further comprising stratifying said subject,
   (i) wherein an increase in the level of BD-tau of about 0.9 pg/ml/h or higher is equivalent to an ASPECTS of 0-2,
   (ii) wherein an increase in the level of BD-tau between about 0.5 to 0.9 pg/ml/h is equivalent to an ASPECTS of 3-5,
   (iii) wherein an increase in the level of BD-tau between about 0.1 to 0.5 pg/ml/h is equivalent to an ASPECTS of 6-8,
   (iv) wherein an increase in the level of BD-tau up to about 0.1 pg/ml/h is equivalent to an ASPECTS of 9-10,
   wherein the stratification allows for the selection of said subject for an appropriate treatment and/or the prediction of outcome.
Item 12: the method of any one of items 2 to 10, further comprising stratifying said subject,
   (i) wherein an increase in the level of BD-tau of about 0.9 pg/ml/h or higher is equivalent to a collateral grade 0,
   (ii) wherein an increase in the level of BD-tau between about 0.5 to 0.9 pg/ml/h is equivalent to a collateral grade 1,
   (iii) wherein an increase in the level of BD-tau between about 0.1 to 0.5 pg/ml/h is to a collateral grade 2,
   (iv) an increase in the level of BD-tau up to about 0.1 pg/ml/h is equivalent to a collateral grade 3,
   wherein the stratification allows for the selection of said subject for an appropriate treatment and/or the prediction of outcome.
Item 13: the method of item 11 or 12, wherein said treatment is intravenous thrombolysis, endovascular thrombectomy, and/or administration of a suitable agent.
Item 14: the method of item 13, wherein said suitable agent is a neuroprotective agent.
Item 15: the method of any one of the preceding items, wherein said blood sample contains serum or plasma.
Item 16: use of the level of BD-tau quantified in a blood sample obtained from a subject suspected to or suffering from a condition causing brain injury for determining the potential extent of brain injury in said subject.
Item 17: the use of item 16, wherein the higher the level of BD-tau in comparison to the level of BD-tau of a healthy subject is, the greater is the potential extent of brain injury of said subject.
Item 18: use of the increase in the level of BD-tau quantified in a blood sample obtained from a subject suspected to or suffering from a condition causing brain injury for determining the progression of the potential extent of brain injury in a subject.
Item 19: the use of item 18, wherein an increase in the level of BD-tau over the course of time is indicative of the progression of the potential extent of brain injury of said subject.
Item 20: the use of any one of items 16 to 19, wherein said brain injury is acute brain injury.
Item 21: the use of any one of items 16 to 20, wherein said subject is suspected to or suffers from a condition causing brain injury.
Item 22: the use of any one of items 16 to 21, wherein said condition causing brain injury is stroke, preferably ischemic stroke, e.g. acute ischemic stroke.
Item 23: a kit comprising an antibody specific for BD-tau and at least two containers for storing and/or processing blood samples.
Item 24: the kit for the use of item 23, further comprising an antibody directed against N-terminal tau.
Item 25: the kit of items 23 or 24 for use in the method of any one of items 1 to 15.

### EXAMPLES

The following examples illustrate and demonstrate the present invention, but do not limit the same.

### Example 1: Study population

The enrollment and eligibility of patients is described in Method 2. 502 patients with ischemic stroke, 51 patients with stroke mimics, and 102 healthy controls were included for analysis. For patients with ischemic stroke, BD-tau levels were available for all 502 patients upon admission and on day 2 (100%), for 498 patients on day 3 (99.2%), 500 patients on day 7 (99.6%), and for 124 patients at day 90 (24.7%). Data on functional outcome was obtained at day 7 on 497 patients (99.0%) and at day 90 on 363 patients (72.3%). Infarct size data were available on 487 patients (97.0%).

The median age of patients with ischemic stroke was 76 years and 41% of these patients were women. Patients were admitted after a median time from onset of 4.4 hours (interquartile range [IQR], 2.1 to 7.7). The median National Institutes of Health Stroke Scale (NIHSS) score at baseline was 6 (IQR, 3 to 13) and the median infarct size was 9.4 ml (IQR, 1.9 to 36.7). The sample was representative of patients presenting with acute ischemic stroke with regards to age and stroke severity.

### Example 2: Temporal course of BD-tau after ischemic stroke

In patients with acute ischemic stroke, BD-tau levels increased from admission (median, 2.9 pg/ml; IQR, 1.8 to 4.8) to day 2 (median, 5.0 pg/ml; IQR, 2.6 to 10.3; P<0.0001). They continued to rise at day 3 (median, 6.1 pg/ml; IQR, 3.2 to 15.0) and day 7 (median, 7.2 pg/ml; IQR, 3.4 to 20.2) followed by a decrease at day 90 (median, 2.2 pg/ml; IQR, 1.5 to 3.0; between any two time points: P<0.0001). Patients with ischemic stroke showed higher BD-tau levels upon admission, at day 2, day 3, and day 7 compared with healthy controls (median, 1.8 pg/ml; IQR, 1.3 to 1.9; all P<0.0001), also when adjusting for age and sex (see Table 1; all P<0.0001). Compared to patients with stroke mimics, BD-tau levels in patients with ischemic stroke were significantly higher at day 2, day 3, and day 7 (all P<0.05; Fig. 1A), also when adjusting for age and sex (all P<0.05).

**Table 1. Relation of BD-tau levels over time with confounding factors**

| **Characteristic** | **Day 1** | **Day 2** | **Day 3** | **Day 7** | **ΔBD-tau** |
|---|---|---|---|---|---|
| Age | <0.0001 | <0.0001 | <0.0001 | <0.0001 | 0.054 |
| Sex | 0.049 | 0.64 | 0.76 | 0.82 | 0.41 |
| Hypertension | 0.0006 | 0.018 | 0.026 | 0.048 | 0.71 |
| Diabetes mellitus Type 2 | 0.0001 | 0.001 | 0.01 | 0.069 | 0.15 |
| Atrial fibrillation | 0.0006 | 0.015 | 0.005 | 0.013 | 0.79 |
| Glomerular filtration rate | <0.0001 | <0.0001 | <0.0001 | <0.0001 | 0.028 |

Shown are P values for the relation of BD-tau levels at individual time points after stroke with different patient characteristics. P values were calculated using t-tests and linear models. BD-tau, brain-derived tau.

To capture the real-time evolution of BD-tau levels during acute stroke and particularly to determine when they start to rise, the inventors sampled two patients every hour from admission until 36 hours after onset (details in Method 9): a rise in BD-tau levels was observed from the first assessments three and five hours after onset. BD-tau levels then progressed at different rates and patterns: while in one patient continuously increasing until at least 48 hours post-onset, BD-tau levels reached a plateau twelve hours after onset in the second patient followed by a secondary increase, which preceded clinical detection of neurological deterioration and the diagnosis of secondary intracerebral hemorrhage (Fig. 5).

### Example 3: Relation of BD-tau to imaging-based metrics of brain injury

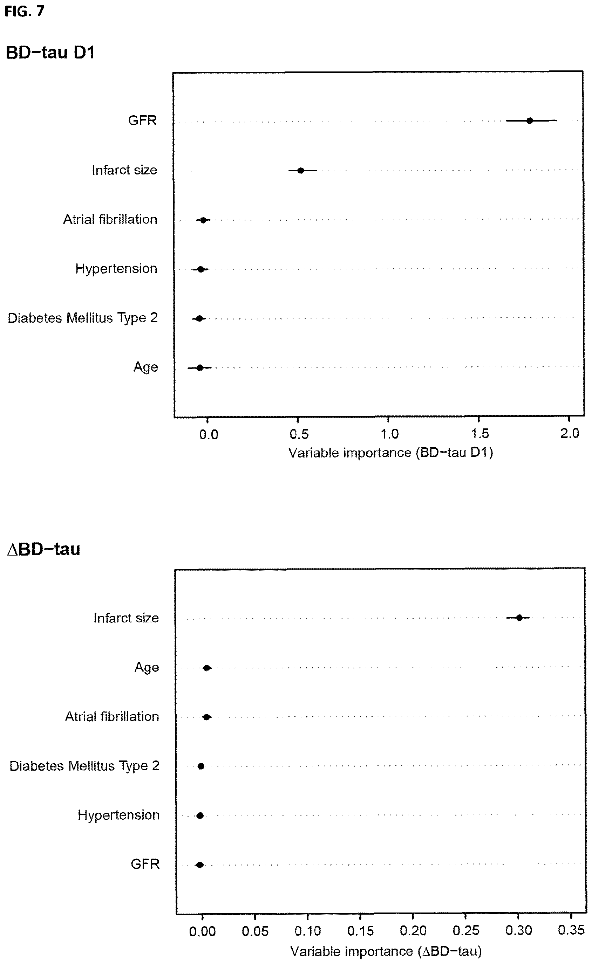

In multivariable random forest regression, the change of BD-tau between admission (day 1) and day 2 (ΔBD-tau) was almost exclusively explained by infarct size, rather than patients' baseline characteristics including age. This was in contrast to absolute BD-tau levels upon admission (Fig. 7). ΔBD-tau was significantly correlated with infarct size (*ρ*=0.604, P<0.0001; Fig. 2A), also when restricting the analysis to those patients with infarct size quantification based on follow-up MRI (N=288 [57%], *ρ*=0.563, P<0.0001). ΔBD-tau was not significantly related to patients' age, status of hypertension, atrial fibrillation as well as glomerular filtration rate (all P>0.05), in contrast to absolute BD-tau levels from admission to day 7 after stroke.

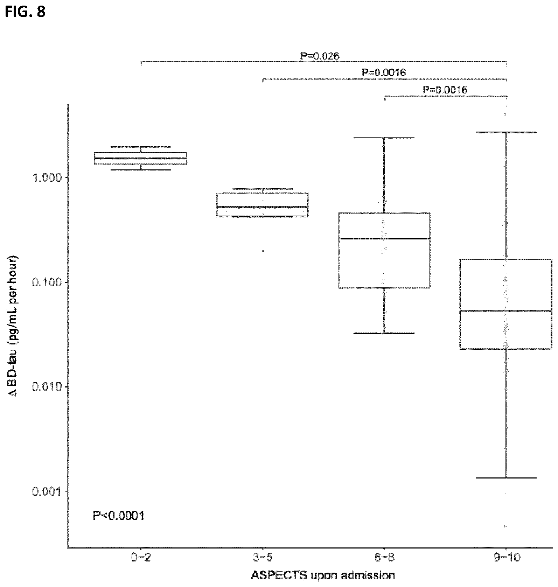

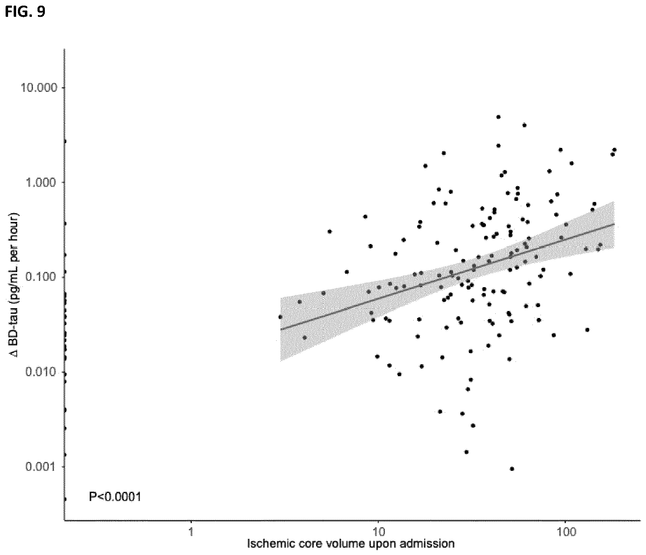

In analyses relating ΔBD-tau to admission CT-based metrics of brain injury, ΔBD-tau was significantly correlated with non-contrast CT-based ASPECTS upon admission (*ρ*=-0.379, P<0.0001; Fig. 8) and CT perfusion-based ischemic core volume (*ρ*=0.477, P<0.0001; Fig. 9). ΔBD-tau was higher in patients with lower collateral supply upon admission (*R*²=0.12, P<0.0001; Fig. 2B), also in analysis adjusted for ischemic core volume (adjusted *R*²=0.11, P<0.0001). ΔBD-tau showed considerably higher value for predicting infarct size compared with non-contrast CT-based ASPECTS and CT perfusion-based ischemic core volume (Fig. 2C).

### Example 4: Prediction of functional outcome after stroke

Higher ΔBD-tau was significantly associated with higher mRS scores (i.e. worse functional outcome) at day 7 (OR, 46.2; 95% Cl, 13.8 to 154.4; P<0.0001; Fig. 3A), also when adjusting for age, sex, hypertension, and premorbid mRS (adjusted OR [aOR], 43.5; 95% Cl, 11.9 to 158.3; P<0.0001). Higher ΔBD-tau was also significantly associated with higher mRS scores at day 90 (OR, 20.6; 95% Cl, 5.4 to 78.7; P<0.0001; Fig. 3B), also in adjusted analysis (aOR, 16.8; 95% Cl, 4.0 to 69.3; P=0.0001). Similar results were found for the rate of functional independence at day 7 and day 90 in both univariable and multivariable analyses (all P<0.01, Figs. S10 and S11). In multivariable random forest regression, ΔBD-tau showed higher value in predicting functional outcome compared with age, sex, premorbid mRS, and admission CT-based ASPECTS and ischemic core volume (Fig. 3C).

The following methods were used:

### Method 1: Participant screening and procedures intended to ensure a representative sample of eligible patients

Patients were recruited in the emergency department of the LMU University Hospital, a comprehensive stroke center in Munich, Germany, between October 2013 and February 2021. Patients were identified by two independent and partially overlapping mechanisms: First, during working hours, research personnel continuously screened the hospital's clinical database for patients admitted with acute stroke-like symptoms to the emergency department. Second, hospital staff informed research personnel both during working hours and non-working hours of eligible patients admitted with acute stroke-like symptoms to the emergency department. Thus, recruitment occurred continuously, 24 hours a day, seven days a week. Further, screening procedures covered both patients directly admitted to LMU University Hospital and patients referred from a primary stroke center. To prevent a bias towards mildly affected patients, a deferred consent process was used for those initially unable to provide consent and when a legally authorized representative was not available (see Method 5).

### Method 2: Inclusion and exclusion criteria

Patients were eligible if they presented with rapidly developing clinical signs suggestive of stroke within 24 hours of symptom onset and were at least 18 years of age. The final diagnosis of ischemic stroke was based on the presence of a diffusion weighted imaging (DWI)-positive lesion on MRI or a new lesion on a delayed CT scan. Patients without a DWI-positive lesion on MRI or a new lesion on a delayed CT scan were classified as having stroke mimics. Patients were excluded if they had no follow-up CT or MRI imaging, underwent major surgery or experienced myocardial infarction, ischemic stroke, transient ischemic attacks, traumatic brain injury, cerebral venous sinus thrombosis, any intracranial hemorrhage, thrombosis, or pulmonary embolism within the four weeks prior to admission, suffered from an in-house stroke, were comorbid for chronic inflammatory bowel disease, or had undergone percutaneous endoscopic gastrostomy. Patients could only be enrolled once in the study. Among initially eligible patients, those who had blood samples collected upon admission (day 1), the next morning (day 2), day 3, and day 7 (or discharge if earlier) were selected.

### Method 3: Collection and management of data including of functional outcome at follow-up

During patients' hospitalization, research personnel reviewed patients' hospital charts and ensured the collection of blood samples. For unwitnessed onset stroke, time of onset was considered to be the midpoint between last seen well and time of recognition. Functional outcome of patients with ischemic stroke was evaluated at day 7 (or discharge if earlier) and at day 90 using the modified Rankin Scale (mRS) score ranging from 0 (no symptoms) to 6 (death). At day 90, the mRS was assessed either by face-to-face or telephone interview. mRS scores were assessed by trained neurologists. All clinical information of recruited patients was assessed independently by two members of the study team and finally reviewed by the investigator.

### Method 4: Recruitment of healthy controls

Healthy controls (HC) were recruited through a single outpatient clinic at LMU University Hospital. These were mostly spouses or companions of patients of the outpatient clinic. Compared to the recruited cohort of patients with ischemic stroke (IS), the cohort of initially included 247 healthy controls comprised more women (HC: 156 women [63%] vs. IS: 208 women [41%], P<0.0001) and was younger (HC: median age [IQR]: 68 years [55-75] vs IS: 76 [66-83], P<0.0001). Selecting those healthy controls with an age of 66 years or higher (N=102), largely eliminated those differences (sex: HC: 44% vs. IS: 41%, P=0.66; age: HC: 75 years (71-78) vs. IS: 76 [66-83], P=0.84).

### Method 5: Deferred consent

A deferred consent process was used for those patients unable to provide consent (e.g. patients with aphasia) and when a legally authorized representative was not available, allowing collection of plasma samples while awaiting consent.

### Method 6: Blood sampling and processing

Blood samples were collected upon hospital admission in the emergency department (day 1), at the next morning (day 2), at day 3, and at day 7. Additional samples were collected at day 90 during follow-up for a subset of patients with ischemic stroke. Blood samples at day 2, 3, and 7 were collected in the morning in parallel to routine blood tests if possible. Whole blood was drawn into EDTA-plasma containers (Sarstedt) by venipuncture or from existing arterial or venous lines. After 30 to 45 minutes at room temperature, separation of plasma was achieved by differential centrifugation at 2000g for 10 minutes at 15 °C. Samples were aliquoted in screw cap vials and kept at -80°C.

### Method 7: BD-tau measurements

Plasma BD-tau was quantified on the Simoa HD-X platform (Quanterix) at the University of Gothenburg, Mölndal, Sweden using a standard protocol well known to the scientific community. In short, the sheep monoclonal antibody TauJ.5H3 (Bioventix Plc, Surrey, UK) was used for capture and an N-terminal-tau mouse monoclonal antibody was used for detection. Recombinant full-length tau-441 (TO8-50FN, SignalChem) was used as calibrator. Assay development and validation procedures, including dilution linearity and spike recovery, have recently been described (Gonzalez-Ortiz, Turton, *et al.,* 2023). Dilution factor was one in four. Six samples were diluted one in ten to fall within the range of the standard curve. Intra- and inter-run repeatability was calculated using two internal quality controls: variation in the whole cohort was <10% (Table 2, Fig. 4). Experimenters were blinded for patient characteristics including group allocation and imaging-based metrics of brain injury.

**Table 2. Concentrations of plasma BD-tau in quality controls**

| | Quality control 1 (n = 76) | Quality control 2 (n =78) |
|---|---|---|
| Mean concentration (pg/ml) | 3.7 ±1.32 | 1.2 ±0.46 |
| Repeatability (% CV) | 8.5 | 9.1 |

| | | |
|---|---|---|
| CV: coefficient of variation | | |

### Method 8: Neuroimaging studies and assessment of brain injury

Multimodal CT was obtained as part of clinical routine upon hospital admission using a standard protocol that included non-contrast CT, CT angiography, and CT perfusion. CT examinations were performed on SOMATOM Definition Force, AS + and Flash scanners (Siemens Healthineers, Forchheim, Germany). CT perfusion data were processed using syngo Neuro Perfusion CT (Siemens Healthineers, Forchheim, Germany) including threshold-based calculation of the ischemic core (cerebral blood volume <1.2 ml/100 ml). ASPECTS on non-contrast CT and the collateral score on CT angiography were assessed by experienced radiologists. Admission CT-based metrics of acute brain injury were quantified in those patients with complete multimodal CT imaging protocol including non-contrast CT, CT angiography, and CT perfusion and thus restricted to 220 patients (43.8%). Infarct size was quantified on images from delayed diagnostic scans (at least 48 hours after stroke onset), either CT or MRI (diffusion-weighted, T2 or fluid-attenuated inversion recovery). The modality and image with the largest infarct size was used for volumetry. Trained raters segmented infarcts manually slice-by-slice. All raters were blinded for BD-tau levels.

### Method 9: Serial high-frequency sampling to assess real-time evolution of BD-tau after stroke onset

To capture the real-time evolution of BD-tau levels within the first 24 hours after onset, two patients with ischemic stroke were sampled with high frequency from admission (three and five hours after onset) until at least 36 hours after onset. Legally authorized representatives gave informed consent in accord with ethical approval. Plasma samples were collected hourly, except between 3AM and 5AM in the morning and during diagnostic procedures. Plasma samples were processed as described in Method 6.

### Method 10: Statistical analyses

Differences in patient characteristics were assessed using the Kruskal-Wallis rank sum test, Pearson's Chi-squared test and the Fisher's exact test. To test for normality, the inventors used the Shapiro-Wilk test. If significant, log transformation (base e) was used. Linear mixed models were used ('Ime4' package in R) to test the temporal course of BD-tau in ischemic stroke. Standardized beta and 95% confidence intervals (CI) are reported. Comparisons between ischemic stroke, stroke mimics and healthy controls were tested with linear models, adjusted for age and sex and post-hoc Tukey HSD tests (corrected for multiple testing). The difference between relative changes of BD-tau and NfL levels in percentage in the acute phase were tested with a paired-sample t-test. The change in BD-tau levels from admission to day 2 was calculated and corrected for the hours between sampling on admission and day 2 for each individual patient and termed ΔBD-tau. To determine the relation between ΔBD-tau and imaging-based metrics of brain injury, we used spearman correlation for continuous variables and analysis for variance for categorical variables. To determine the relationship between ΔBD-tau and the mRS score at 7 and 90 days after stroke (as dependent variable) the inventors used ordinal (for the mRS score distribution) and binomial (for the rate of functional independence defined as an mRS score of 0-2) univariable and multivariable logistic regression adjusting for potential baseline confounder variables (age, sex, hypertension, pre-stroke mRS). Odds ratios (OR) and 95% CI are reported. A P value <0.05 was considered statistically significant. Random forest regression was used to assess conditional variable importance in multivariable models for the dependent variables BD-tau (day 1 and ΔBD-tau), infarct size and functional outcome (day 7 and day 90).

Linear mixed models were used to test the temporal course of BD-tau: first to test differences between the different time points (fixed effect: days, random effect: patient ID) and second to test the interaction between hours since symptom onset and infarct volume (fixed effects) with patient ID as random effect. While plots displaying ΔBD-tau values are restricted to positive values on the log-scaled axis, statistical analyses include all available values. Associations between BD-tau and age, sex, hypertension, diabetes mellitus type 2, atrial fibrillation, and the glomerular filtration rate were tested with Mann-Whitney U tests for categorical and linear models for continuous variables. To assess conditional variable importance in multivariable models we used random forest regression with standard parameters, grew 1,501 trees per run, and performed 100 runs to calculate 95% confidence intervals ('party' package in R). For all analyses, complete case analysis was performed without imputations.

### REFERENCES

Baig, M.U. and Bodle, J. (2023) 'Thrombolytic Therapy', in StatPearls. Treasure Island (FL): StatPearls Publishing. Available at: http://www.ncbi.nlm.nih.gov/books/NBK557411/ (Accessed: 5 September 2023).
Barber, P.A. et al. (2000) 'Validity and reliability of a quantitative computed tomography score in predicting outcome of hyperacute stroke before thrombolytic therapy. ASPECTS Study Group. Alberta Stroke Programme Early CT Score', Lancet (London, England), 355(9216), pp. 1670-1674. Available at: https://doi.org/10.1016/s0140-6736(00)02237-6.
Boers, A.M.M. et al. (2019) 'Mediation of the Relationship Between Endovascular Therapy and Functional Outcome by Follow-up Infarct Volume in Patients With Acute Ischemic Stroke', JAMA neurology, 76(2), pp. 194-202. Available at: https://doi.org/10.1001/jamaneurol.2018.3661.
Ghozy, S. et al. (2022) 'Neuroprotection in Acute Ischemic Stroke: A Battle Against the Biology of Nature', Frontiers in Neurology, 13, p. 870141. Available at: https://doi.org/10.3389/fneur.2022.870141.
Gonzalez-Ortiz, F., Turton, M., et al. (2023) 'Brain-derived tau: a novel blood-based biomarker for Alzheimer's disease-type neurodegeneration', Brain: A Journal of Neurology, 146(3), pp. 1152-1165. Available at: https://doi.org/10.1093/brain/awac407.
Gonzalez-Ortiz, F., Dias, A., et al. (2023) 'Preanalytical stability of plasma/serum brain-derived tau', Alzheimer's & Dementia: The Journal of the Alzheimer's Association [Preprint]. Available at: https://doi.org/10.1002/alz.13156.
Ki 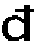 emet-Piskač, S. *et al.* (2018) 'Evaluation of cerebrospinal fluid phosphorylated tau231 as a biomarker in the differential diagnosis of Alzheimer's disease and vascular dementia', *CNS Neuroscience & Therapeutics,* 24(8), pp. 734-740. Available at: https://doi.org/10.1111/cns.12814.
Oliveira, A.J.F., Viana, S.M.N. and Santos, A.S. (2022) 'Mechanical thrombectomy for acute ischemic stroke: systematic review and meta-analysis', Einstein (Sao Paulo, Brazil), 20, p. eRW6642. Available at: https://doi.org/10.31744/einstein_journal/2022RW6642.

## Claims

1. A method for determining the potential extent of brain injury in a subject, comprising quantifying in a blood sample obtained from said subject the level of BD-tau, wherein the higher the level of BD-tau in comparison to the level of BD-tau of a healthy subject is, the greater is the potential extent of brain injury of said subject.

2. A method for determining the progression of the potential extent of brain injury in a subject, comprising quantifying in a blood sample obtained from said subject over the course of time the level of BD-tau, wherein an increase in the level of BD-tau over the course of time, is indicative of the progression of the potential extent of brain injury of said subject.

3. The method of any one of the preceding claims, wherein said brain injury is acute brain injury.

4. The method of any one of the preceding claims, wherein said subject is suspected to or suffers from a condition causing brain injury.

5. The method of any one of the preceding claims, wherein said condition causing brain injury is stroke, preferably ischemic stroke, e.g. acute ischemic stroke.

6. The method of any one of the preceding claims, wherein a level of BD-tau of about 1.8 pg/ml or more is indicative of the potential extent of brain injury of said subject.

7. The method of any one of claims 2 to 6, wherein an increase of about 0.01 pg/ml/h or more is indicative of the potential progression of the extent of brain injury.

8. The method of any one of claims 2 to 7, further comprising stratifying said subject,
(i) wherein an increase in the level of BD-tau of about 0.9 pg/ml/h or higher is equivalent to an ASPECTS of 0-2,
(ii) wherein an increase in the level of BD-tau between about 0.5 to 0.9 pg/ml/h is equivalent to an ASPECTS of 3-5,
(iii) wherein an increase in the level of BD-tau between about 0.1 to 0.5 pg/ml/h is equivalent to an ASPECTS of 6-8,
(iv) wherein an increase in the level of BD-tau up to about 0.1 pg/ml/h is equivalent to an ASPECTS of 9-10,
wherein the stratification allows for the selection of said subject for an appropriate treatment and/or the prediction of outcome.

9. The method of any one of claims 2 to 7, further comprising stratifying said subject,
(i) wherein an increase in the level of BD-tau of about 0.9 pg/ml/h or higher is equivalent to a collateral grade 0,
(ii) wherein an increase in the level of BD-tau between about 0.5 to 0.9 pg/ml/h is equivalent to a collateral grade 1,
(iii) wherein an increase in the level of BD-tau between about 0.1 to 0.5 pg/ml/h is to a collateral grade 2,
(iv) an increase in the level of BD-tau up to about 0.1 pg/ml/h is equivalent to a collateral grade 3,
wherein the stratification allows for the selection of said subject for an appropriate treatment and/or the prediction of outcome.

10. The method of claim 8 or 9, wherein said treatment is intravenous thrombolysis, endovascular thrombectomy, and/or administration of a suitable agent.

11. Use of the level of BD-tau quantified in a blood sample obtained from a subject suspected to or suffering from a condition causing brain injury for determining the potential extent of brain injury in said subject,
wherein the higher the level of BD-tau in comparison to the level of BD-tau of a healthy subject is, the greater is the potential extent of brain injury of said subject.

12. Use of the increase in the level of BD-tau quantified in a blood sample obtained from a subject suspected to or suffering from a condition causing brain injury for determining the progression of the potential extent of brain injury in a subject,
wherein an increase in the level of BD-tau over the course of time is indicative of the progression of the potential extent of brain injury of said subject.

13. A kit comprising an antibody specific for BD-tau and at least two containers for storing and/or processing blood samples.

14. The kit for the use of claim 13, further comprising an antibody directed against N-terminal tau.

15. The kit of claims 13 or 14 for use in the method of any one of claims 1 to 10.
